# EUROPEAN PATENT APPLICATION

(11) **EP 4 722 688 A1**
(43) Date of publication of application: **08.04.2026**
(21) Application number: 24814567.4
(22) Date of filing: 31.05.2024
(51) Int. Cl.: G01N 1/38, C12M 1/00, G01N 35/00, G01N 33/00

(54) **TREATMENT DEVICE FOR SAMPLE**

(30) Priority: 31.05.2023 CN 202310636690
(71) Applicant: Nanjing GenScript Biotech Co., Ltd., Nanjing, Jiangsu 211100 (CN)
(72) Inventor: WEI, Jinwen, Nanjing, Jiangsu 211100 (CN); SUN, Zhenbo, Nanjing, Jiangsu 211100 (CN); ZHENG, Cheng, Nanjing, Jiangsu 211100 (CN); FANG, Jiujin, Nanjing, Jiangsu 211100 (CN); ZHOU, Yang, Nanjing, Jiangsu 211100 (CN); ZHANG, Zhun, Nanjing, Jiangsu 211100 (CN); QIAO, Yuehua, Nanjing, Jiangsu 211100 (CN); KE, Lanlan, Nanjing, Jiangsu 211100 (CN)
(74) Representative: Latscha Schöllhorn Partner AG
(86) International application number: PCT/CN2024/096523
(87) International publication number: WO 2024/245372

(57) **Abstract**

A processing device for a sample, the processing device including: a magnetic attraction and mixing mechanism, including a mixing assembly and a magnetic assembly for respectively performing mixing and magnetic attraction on the sample; and a processing box, including two sets of processing chambers spaced apart from each other. One of the two sets of processing chambers is configured to process the sample at the same moment.

## Description

### Cross Reference to Related Application

The present invention claims priority to Chinese Patent Application No. 202310636690.9 filed on May 31, 2023, which is incorporated herein by reference in its entirety.

### Technical Field

The present description relates to the technical field of biological devices, more particularly, to a processing device for a sample.

### Background Art

Extraction or purification of target biological substances (e.g., plasmids) is a crucial step in molecular biology and medical research, and the performance of extraction or purification directly affect the progress and results of research and diagnosis. In modern bioengineering, plasmids are used as vectors for genetic engineering. DNA fragments are inserted into plasmids by experimenters, and then replicated in large quantities by means of bacteria, and finally the plasmids are extracted from the bacteria. The main steps of the extraction or purification process of a target biological substance (e.g., plasmid) include adding specific reagents into a sample to achieve different functions (e.g., lysis reaction with the sample, or separation of magnetic beads from a magnetic bead conjugate) and adding magnetic beads to the sample, such that the magnetic beads are conjugated to impurities or the target biological substance, in order to separate the impurities or the target biological substance from the solution. The mixing efficiency of the reagents and the sample, and the conjugation efficiency of the magnetic beads and the impurities or the target biological substance will affect the effectiveness of extraction or purification of the entire target biological substance (e.g., plasmid).

Therefore, it is necessary to provide a processing device for a sample to effectively improve the mixing efficiency of a reagent and the sample, and the conjugation efficiency of magnetic beads and impurities or a target biological substance.

### Summary

One of the embodiments of the present description provides a processing device for a sample, the processing device including: a magnetic attraction and mixing mechanism, including a mixing assembly and a magnetic assembly for respectively performing mixing and magnetic attraction on the sample; and a processing box, including two sets of processing chambers.

### Brief Description of the Drawings

The present description will be further illustrated by way of exemplary embodiments, and these exemplary embodiments will be described in detail with reference to the accompanying drawings. These embodiments are not limiting, and in these embodiments, the same reference numerals represent the same structures, in which:
Fig. 1 is a schematic structural view of a processing device according to some embodiments of the present description;
Fig. 2 is a schematic structural view of a processing box according to some embodiments of the present description;
Fig. 3 is a schematic structural view of the processing box connected to a mixing assembly according to some embodiments of the present description;
Fig. 4 is a schematic cross-sectional view of the processing box connected to the mixing assembly according to some embodiments of the present description;
Fig. 5 is a schematic cross-sectional view of the processing box connected to the mixing assembly according to some other embodiments of the present description;
Fig. 6 is a schematic structural view of the processing box connected to a magnetic assembly according to some embodiments of the present description; and
Fig. 7 is a schematic cross-sectional view of the processing box connected to the magnetic assembly according to some embodiments of the present description.

Reference signs: processing device 100; magnetic attraction and mixing mechanism 10; magnetic assembly 11; magnet driving mechanism 110; first driving motor 111; lead screw 112; linkage 113; magnet support member 114; linear guide rail 1141; base 1142; lead screw nut 115; nut track 1151; mixing assembly 12; second driving motor 121; motor fixing plate 1211; mixing platform 122; eccentric shaft 1221; first mixing bearing 1231; second mixing bearing 1232; third mixing bearing 1233; first direction adapter plate 1241; second direction adapter plate 1242; first mixing guide rail 1251; second mixing guide rail 1252; support plate 126; connecting shaft 127; coupling 128; second optocoupler barrier 1291; second optocoupler 1292; processing box 20; processing chamber 21; first processing chamber 211; second processing chamber 212.

### Detailed Description of Embodiments

For a clear description of the technical solutions of the embodiments in the present description, the accompanying drawings required for describing the embodiments will be briefly described below. Apparently, the accompanying drawings in the following description show merely some examples or embodiments of the present description, and those of ordinary skill in the art may still apply the present description to other similar scenarios according to these accompanying drawings without any creative effort. Unless obvious from the linguistic context or otherwise stated, the same reference signs in the drawings represent the same structure or operation.

As shown in the present description and the claims, the words "one", "a", "an" and/or "the" do not specifically refer to the singular, but may also include the plural, unless the context clearly indicates otherwise. Generally, the terms "comprising" and "including" only elicit explicitly identified steps and elements, which, however, are not exclusive in the list. In other words, said method or device may further include other steps or elements.

The present description provides a processing device for a sample. The processing device may include a processing box for containing the sample, and a magnetic attraction and mixing mechanism. The magnetic attraction and mixing mechanism may be configured to perform magnetic attraction and mixing on the sample in the processing box. In some embodiments, the processing device may be used in conjunction with an extraction apparatus for a sample to extract and/or purify a target biological substance in a sample, so that it is possible to significantly reduce the workload of experimenters and shorten the work time. Moreover, the processing device is mechanically automated throughout its work, with high consistency in repeated operations and better overall extraction and/or purification results. In some embodiments, the processing device can be used for high-throughput sample processing, i.e., performing extraction and/or purification on a plurality of samples simultaneously, to significantly improve the efficiency of extraction and/or purification. The target biological substance in the present description is a substance in the sample that is to be extracted (or separated) and/or purified. In some embodiments, the target biological substance includes a plasmid (e.g., a DNA plasmid, or an RNA plasmid), a protein, a nucleic acid, and any other feasible biological substances. For example, the processing device can be used to process a sample to extract and/or purify a plasmid. For another example, the processing device can be used to process a sample to extract and/or purify a protein. In some embodiments, the sample may be a microorganism (including bacteria, viruses, fungi, etc. such as escherichia coli) culture solution, blood, saliva, an animal tissue, a plant, food, or any other feasible samples. Extraction is the separation of the target biological substance from other impurities in a sample (e.g., a culture solution). Purification is the further purification of the separated target biological substance. It should be understood that application scenarios of the processing device in the description of the present application are merely some examples or embodiments of the present application. Those of ordinary skill in the art may still apply the present application to other similar scenarios according to the accompanying drawings without any creative effort.

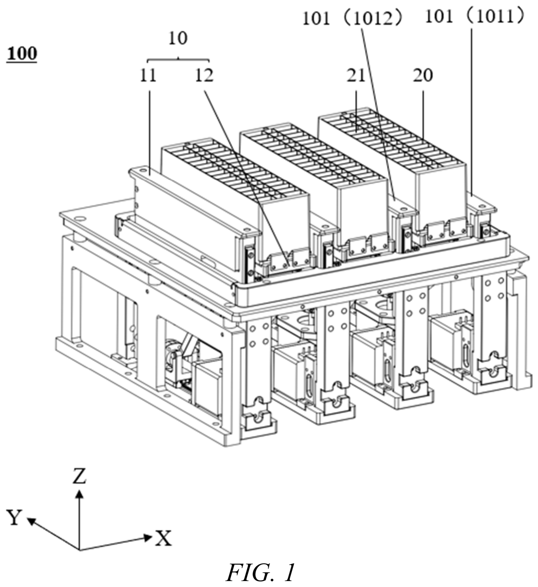

Fig. 1 shows a schematic structural diagram exemplarily showing a processing device 100 for a sample. For ease of description, the various assemblies and/or devices of the processing device 100 are further described using a coordinate system as shown in Fig. 1. The coordinate system (e.g., a Cartesian coordinate system) includes an X-axis, a Y-axis, and a Z-axis. The Z-axis is parallel to a vertical direction (for example, being perpendicular to the ground), the X-axis and the Y-axis are parallel to a horizontal direction (for example, being parallel to the ground), and the X-axis is perpendicular to the Y-axis. The Y-axis direction is also referred to as a first direction, the X-axis direction is also referred to as a second direction, and the Z-axis direction is also referred to as a third direction.

In some embodiments, the processing device 100 may include a magnetic attraction and mixing mechanism 10 and a processing box 20. The magnetic attraction and mixing mechanism 10 may include a mixing assembly 12 and a magnetic assembly 11. The mixing assembly 12 may be configured to mix the sample, and the magnetic assembly 11 may be configured to magnetically attract the sample. The processing box 20 may include two sets of processing chambers 21.

In the present description, mixing is to mix a mixture in the processing chamber 21 by shaking or vibration. Magnetic attraction is the use of magnetic attraction action to separate impurities or a target biological substance from the sample. By way of example only, during the process of extracting and/or purifying a sample, it is necessary to add specific reagents into the sample to achieve different functions (e.g., lysis reaction with the sample, or separation of magnetic beads from a magnetic bead conjugate) and add magnetic beads to the sample, such that the magnetic beads are conjugated to impurities or a target biological substance in the sample, in order to separate the target biological substance or impurities from the solution. The magnetic bead in the present description is a magnetic particle that has a surface-active group. Under certain conditions, the surface-active group of the magnetic bead can be conjugated to (e.g., be coupled with) the target biological substance (e.g., a substance to be extracted) or impurities (e.g., substances other than the substance to be extracted) in the sample. Under the action of an external magnetic field (e.g., a magnetic field formed by the magnetic assembly), the magnetic beads can be subjected to magnetic attraction (i.e., magnetically attracted), thereby achieving separation of the active substance from the impurities and/or purification of the active substance.

In some embodiments, the corresponding type of the magnetic bead may be selected depending on the particle size, the dispersity, the magnetic response time, and the coating matrices of the magnetic bead. The type of the magnetic bead is not limited here. In some embodiments, the types of reagents may include solvents for reacting with samples and for washing magnetic bead conjugates, reagents for eluting magnetic bead conjugates to separate magnetic beads, etc. The types of reagents are not limited here. Taking plasmid extraction by means of bacterial sludge as an example, the reagent may include a bacterial sludge lysis solution, a washing solution, an eluent, etc. The magnetic beads may include magnetic beads capable of being conjugated to impurities obtained after bacterial sludge is lysed, magnetic beads capable of being conjugated to a plasmid, etc. In some embodiments, the mixing assembly mixes the magnetic beads with the sample to improve the efficiency of conjugation of the magnetic beads to the impurities and/or the target biological substance in the sample.

In some embodiments, the types of reagents may include solvents for reacting with samples and for washing magnetic bead conjugates, reagents for eluting magnetic bead conjugates to separate magnetic beads, etc. The types of reagents are not limited here. Taking plasmid extraction by means of bacterial sludge as an example, the reagent may include a bacterial sludge lysis solution, a washing solution, an eluent, etc. The magnetic beads may include magnetic beads capable of being conjugated to impurities obtained after bacterial sludge is lysed, magnetic beads capable of being conjugated to a plasmid, etc. In some embodiments, when a reagent is added for reaction with the sample, the mixing assembly 12 may be used to mix the reagent with the sample to improve the efficiency of reaction of the sample. When a reagent is added for washing a magnetic bead conjugate, the mixing assembly 12 may be configured to mix the reagent with the sample to improve the washing efficiency. When a reagent is added for eluting the magnetic bead conjugate to separate the magnetic beads, the mixing assembly 12 may be configured to mix the reagent with the sample to improve the efficiency of separation of the magnetic beads from the magnetic bead conjugate.

The processing device 100 provided in the present description can attract the magnetic beads conjugated to the impurities or the target biological substance in the processing box 20, thereby facilitating the transfer of the remaining solution by a pipetting device so as to separate the impurities or the target biological substance from the solution. In addition, the processing device 100 can mix the mixture in the processing box 20 to improve the efficiency and effectiveness of the extraction and/or purification of the target biological substance.

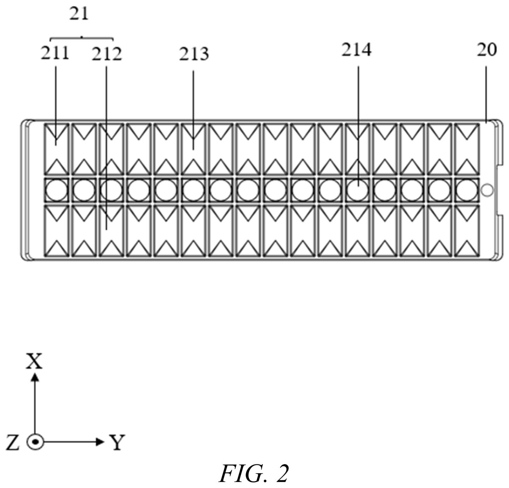

In some embodiments, each set of processing chambers 21 may include a plurality of mounting holes 213. The plurality of mounting holes 213 may be disposed along an extending direction of the processing chambers 21. The mounting hole 213 may be configured to contain the sample. The two sets of processing chambers 21 have the same number of mounting holes 213 which are in one-to-one correspondence. One-to-one correspondence here may mean that in the distribution direction of the two sets of processing chambers 213, any one of the mounting holes 213 in one set of processing chambers 21 is aligned with one of the mounting holes 213 in the other set of processing chambers 21, to facilitate the transfer of the target biological substance from one set of processing chambers 21 to the other set of processing chambers 21 by the pipetting device (not shown). For example, as shown in Fig. 2, the first processing chamber 211 and the second processing chamber 212 each include 16 mounting holes 213. The 16 mounting holes 213 are disposed in the Y-axis direction. In the X-axis direction, one mounting hole 213 of the first processing chamber 211 is aligned with one mounting hole 213 of the second processing chamber 212.

In some embodiments, one of the two sets of processing chambers 21 is used to process the sample at the same moment. In some cases, during the extraction and/or purification of the target biological substance using the processing device 100 provided in the present description, when two processing chambers 21 are used to process the sample simultaneously, due to the need to perform magnetic attraction on the sample in the processing chambers 21 using the magnetic assembly, performing magnetic attraction on the sample in one of the processing chambers 21 may affect the sample in the other processing chamber 21. By way of example only, as shown in Figs. 1 and 2, the magnetic beads in one of the processing chambers (which may be referred to as a first processing chamber, e.g., a first processing chamber 211 in Fig. 2) are conjugated to the impurities, and the magnetic beads in the other processing chamber (which may be referred to as a second processing chamber, e.g., a second processing chamber 212 in Fig. 2) are conjugated to the target biological substance. When the magnetic beads in the first processing chamber 211 are subjected to magnetic attraction, the magnetic beads in the second processing chamber 212 may be attracted, thereby affecting the sample in the second processing chamber 212. Therefore, in order to prevent the sample in the other set of processing chambers 21 from being affected, only one of the two sets of processing chambers 21 is used to process the sample at the same moment.

In some embodiments, the two sets of processing chambers 21 may be spaced apart from each other. In some embodiments, the distance between the two sets of processing chambers 21 may be increased to reduce or avoid the influence on the sample in the other set of processing chambers 21 when performing magnetic attraction, so that the two sets of processing chambers 21 may be used to process the sample simultaneously, to improve the sample processing efficiency. For example, a gap of a preset distance may be provided between the two sets of processing chambers 21. In some embodiments, an isolation member 214 may be provided between the two sets of processing chambers 21 to reduce or block the magnetic force between the two sets of processing chambers 21 so that the two sets of processing chambers 21 may be used to process the sample simultaneously. In some embodiments, the isolation member 214 may include a magnetic shielding plate made of pure iron, an iron-nickel alloy, etc. In some embodiments, the isolation member 214 may not be a magnetic shielding member but physically separate the two sets of processing chambers 21. In some other embodiments, the two sets of processing chambers 21 may be disposed adjacent to each other.

It should be noted that the description of the number of processing boxes 20, the number of processing chambers 21 contained by the processing box 20, and the number of mounting holes 213 is for illustrative purposes only and is not intended to limit the number of processing boxes 20, the number of processing chambers 21 contained by the processing box 20 and the number of mounting holes 213 in the present description. For example, the processing box 20 may include only one processing chamber 21, or the processing box 20 may include three processing chambers 21, which are disposed in parallel at intervals. For another example, the number of processing boxes 20 may be one or plural, which can be adjusted according to actual requirements.

In some embodiments, as shown in Figs. 1-2, the magnetic assembly 11 may include a magnet 101. The magnet 101 may be disposed on at least one side of the mixing assembly 12, and the processing box 20 is disposed on the mixing assembly 12. In this embodiment, the magnet 101 may be configured to form an external magnetic field. After the magnetic beads are conjugated to the impurities or the target biological substance in the sample, the magnetic beads in the processing box 20 may be attracted under the action of the external magnetic field, such that the magnetic beads tightly fits against a side wall of the processing chamber 21 to facilitate the transfer of the remaining solution by means of the pipetting device, thereby separating the impurities or the target biological substance from the solution. In some embodiments, the magnet 101 has a predetermined magnetic attraction area. In some embodiments, the magnetic attraction area of the magnet 101 may be the area of a magnetic attraction surface facing the processing box 20 for magnetic attraction.

In some embodiments, the magnet 101 may include a first magnet 1011. The first magnet 1011 may be disposed on one side of the mixing assembly 12. In this embodiment, the first magnet 1011 may be configured to form a first external magnetic field for performing magnetic attraction on the sample in the first processing chamber 211. For example, after the magnetic beads in the first processing chamber 211 are conjugated to the impurities or the target biological substance in the sample, the magnetic beads may be attracted by the first magnet 1011 to separate the impurities or the target biological substance from the solution.

In some embodiments, the magnet 101 may further include a second magnet 1012. The second magnet 1012 may be disposed on the other side of the mixing assembly 12. In this embodiment, the second magnet 1012 may be configured to form a second external magnetic field for performing magnetic attraction on the sample in the second processing chamber 212. For example, after the magnetic beads in the second processing chamber 212 are conjugated to the impurities or the target biological substance in the sample, the magnetic beads may be attracted by the second magnet 1012 to separate the impurities or the target biological substance from the solution.

In some embodiments, the processing box 20 may be disposed between the first magnet 1011 and the second magnet 1012, and the two sets of processing chambers 21 are arranged in the same direction as the first magnet 1011 and the second magnet 1012. By way of example only, the extending directions of the first processing chambers 211 and the second processing chambers 212 may both be parallel to the Y-axis direction, and the first processing chamber 211 and the second processing chamber 212 are arranged spaced apart in the X-axis direction. The first magnet 1011 and the second magnet 1012 are arranged spaced apart in the X-axis direction, the first magnet 1011 is disposed on a side of the first processing chamber 211 away from the second processing chamber 212, and the second magnet 1012 is disposed on a side of the second processing chamber 212 away from the first processing chamber 211.

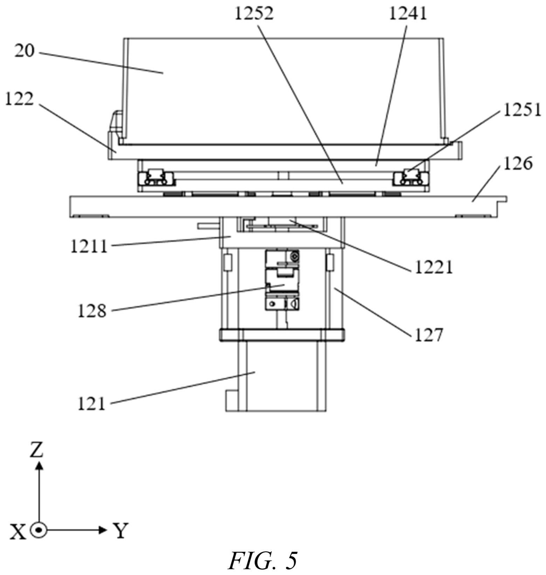

In some embodiments, the number of processing boxes 20 may be one or plural, and the number of mixing assemblies 12 and the number of magnetic assemblies 11 may be adapted to the number of processing boxes 20. By way of example only, one processing box 20 may be subjected to mixing by means of one separate mixing assembly 12 and magnetic attraction by means of two separate magnetic assemblies 11, respectively. One of the magnetic assemblies 11 is configured to drive the first magnet 1011 to extend above or retract below the upper side surface of the magnetic attraction and mixing mechanism 10, and the other magnetic assembly 11 is configured to drive the second magnet 1012 to extend above or retract below the upper side surface of the magnetic attraction and mixing mechanism 10. That is, the number of processing boxes 20 is n, the number of mixing assemblies 12 is n, and the number of magnetic assemblies 11 is 2n. In another example, as shown in Fig. 1, the number of processing boxes 20 is 3, the number of mixing assemblies 12 is 3, the number of magnetic assemblies 11 is 4, the three processing boxes 20 are spaced apart in the X-axis direction, one mixing assembly 12 corresponds to one processing box 20, and the processing boxes 20 are respectively disposed on the mixing platforms (e.g., the mixing platforms 122 in Fig. 5) of the corresponding mixing assemblies 12, the four magnetic assemblies 11 are spaced apart from the processing boxes 20 in the X-axis direction, and two adjacent processing boxes 20 share the same magnetic assembly 11, to reduce the number of magnetic assemblies 11 and reduce the cost of the device. In some embodiments, a plurality of processing boxes 20 may be subjected to mixing by means of the same mixing assembly 12.

It should be noted that categorizing the magnet 101 as the first magnet 1011 and the second magnet 1012 is for illustrative purposes only and is intended to illustrate the positional relationship of the first magnet 1011 and the second magnet 1012 with a processing box 20, and the specific structures, materials, etc. of the first magnet 1011 and the second magnet 1012 are not limited. In some embodiments, the first magnet 1011 and the second magnet 1012 may be the same or similar. For example, the materials, dimensions, etc. of the first magnet 1011 and the second magnet 1012 are the same. In some embodiments, the first magnet 1011 and the second magnet 1012 may be different. In some embodiments, the magnet 101 may act as both the first magnet 1011 and the second magnet 1012. For example, in the embodiment shown in Fig. 1, the magnet 101 located on the right side of the right-most processing box 20 (which may be referred to as the first processing box) in the Y-axis direction may be referred to as the first magnet 1011, and the magnet 101 located on the left side of the first processing box may be referred to as the second magnet 1011, while for an intermediate processing box 20 (which may be referred to as the second processing box), the magnet 101 located on the left side of the first processing box may be referred to as the first magnet 1011.

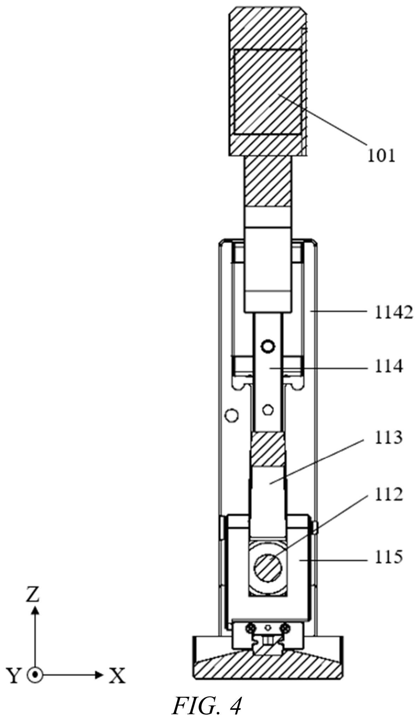

In some embodiments, as shown Figs. 1 and 3-4, the magnetic assembly 11 may further include a magnet driving mechanism 110 for driving the magnet 101 to perform a lifting or lowering movement. The lifting or lowering movement of the magnet 101 is to move the magnet 101 in the vertical direction (i.e., in the Z-axis direction). The magnet driving mechanism 110 driving the magnet 101 to perform the lifting or lowering movement in the vertical direction can enable flexible control over the position of the magnet 101 in the vertical direction. In some embodiments, the number of magnet driving mechanisms 110 may be one or plural. For example, when the magnet 101 includes only the first magnet 1011, the number of magnet driving mechanisms 110 may be one, and the one magnet 101 driving member drives the first magnet 1011 to perform the lifting or lowering movement. For another example, when the magnet 101 includes a first magnet 1011 and a second magnet 1012, the number of magnet driving mechanisms 110 may be two, and the two magnet driving mechanisms 110 drive the first magnet 1011 and the second magnet 1012, respectively, to perform the lifting or lowering movement.

In some embodiments, the magnet driving mechanism 110 may be drivingly connected to the magnet 101, driving the magnet 101 to perform the lifting or lowering movement. In some embodiments, the magnet driving mechanism 110 may include a first driving motor 111, a lead screw 112 and a linkage 113. The lead screw 112 is drivingly connected to the first driving motor 111, one end of the linkage 113 (also called a first end) is hinged to the lead screw 112, the other end of the linkage 113 (also called a second end) is hinged to the magnet 101, and the magnet 101 can be driven by the first driving motor 111 to extend above or retract below an upper side surface of the magnetic attraction and mixing mechanism 10. The upper side surface of the magnetic attraction and mixing mechanism 10 may be a surface of the magnetic attraction and mixing mechanism 10 for carrying the processing box 20. For example, the mixing assembly 12 may include a mixing platform (e.g., a mixing platform 122 in Fig. 5), and the processing box 20 may be disposed on an upper side surface of the mixing platform. After the magnet 101 extends above the upper side surface of the mixing platform, the magnetic beads in the processing box 20 can be attracted. When the magnet 101 is retracted below the upper side surface of the mixing platform, the magnetic beads in the processing box 20 are not affected by the magnet 101.

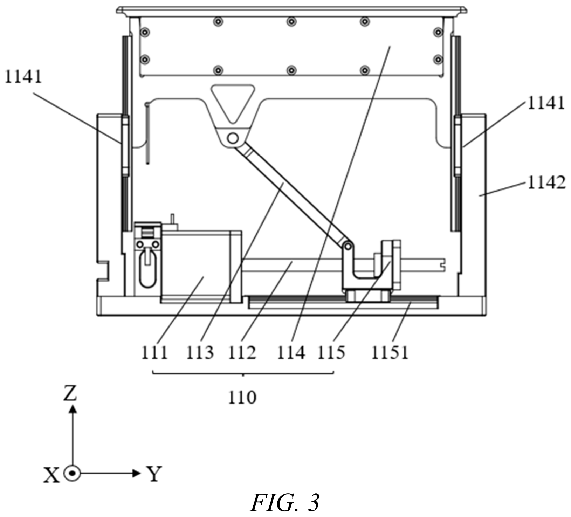

In some embodiments, the second end of the linkage 113 may be hinged directly to the magnet 101. For example, the second end of the linkage 113 and the magnet 101 may each be provided with a hinge, and the second end of the linkage 113 is hinged to the magnet 101 via the hinges. In some embodiments, the second end of the linkage 113 may be hinged indirectly to the magnet 101. For example, the magnet 101 may be disposed on a magnet support member 114 of Fig. 3, and the second end of the linkage 113 is hinged to the magnet support member 114.

In some embodiments, the magnet driving mechanism 110 may include a lead screw nut 115. The lead screw nut 115 is connected between the lead screw 112 and the linkage 113. The lead screw nut 115 is connected to the end of the lead screw 112 away from the first driving motor 111, and the lead screw nut 115 is hinged to the first end of the linkage 113. The lead screw nut 115 may slide on the nut track 1151. An extending direction of the nut track 1151 is the first direction (i.e., the Y-axis direction). That is, the lead screw nut 1151 can move in the first direction. The first driving motor 111 operates to drive the lead screw 112 to rotate, the rotation of the lead screw 112 drives the lead screw nut 115 to move in the first direction, and the movement of the lead screw nut 115 further drives the linkage 113 to rotate in the Y-Z plane, thereby driving the magnet to perform a lifting or lowering movement in the vertical direction (i.e., the Z-axis direction).

In some embodiments, the magnetic assembly 11 may further include a magnet support member 114 for fixing the magnet 101. In some embodiments, the magnet support member 114 may be hinged to the linkage 113. The magnet 101 is fixed to the magnet support member 114, thereby enabling indirect hinging of the second end of the linkage 113 to the magnet.

In some embodiments, an upper end of the magnet support member 114 has a larger dimension in the Y-axis direction, while a lower end of the magnet support member 114 has a smaller dimension in the Y-axis direction. The upper end of the magnet support member 114 may be the end for supporting the magnet 101, and the lower end of the magnet support member 114 may be the end hinged to the linkage 113. The larger dimension of the upper end makes the upper end easier to support the magnet 101, and smaller dimension of the lower end makes the lower end easier to be hinged to the linkage 113.

In some embodiments, a linear guide rail 1141 may be provided on each side of the magnet support member 114. An extending direction of the linear guide rail 1141 is the vertical direction (i.e., the Z-axis direction). The magnet support member 114 can move in the vertical direction on the linear guide rail 1141. By providing the linear guide rail 1141 on each side of the magnet support member 114, it is possible to ensure that the magnet support member 1141 remains balanced when moving in the vertical direction. In some embodiments, the magnet support member 114 may be connected to a base 1142 via the linear guide rails 1141, thereby enabling the magnet support member 114 to move along the linear guide rails 1141 relative to the base 1142. The first driving motor 111 is located below the magnet support member 114, and the lead screw nut 115 is connected to the magnet support member 114 via the linkage 113. The first driving motor 111 operates to drive the lead screw 112 to rotate, the rotation of the lead screw 112 drives the lead screw nut 115 to move in the first direction, and the movement of the lead screw nut 115 further drives the linkage 113 to rotate in the Y-Z plane, thereby driving the magnet support member 114 to move on the linear guide rails 1141 and achieving the lifting or lowering movement of the magnet.

In some embodiments, the magnetic assembly 11 may further include a lifting position sensor which may be configured to determine the position of the magnet 101, for example, to determine whether the magnet 101 is in a first position (i.e., the magnet 101 fully extends above the upper side surface of the magnetic attraction and mixing mechanism 10) or in a second position (i.e., the magnet 101 fully retracts below the upper side surface of the magnetic attraction and mixing mechanism 10). By way of example only, the lifting position sensor may include a first optocoupler and a first optocoupler barrier. One first optocoupler (not shown) is provided at each of the bottom and the top of the base 1142, and the first optocoupler barrier (not shown) is fixed to the magnet support member 114. When the magnet support member 114 moves along the linear guide rails 1141 to make the first optocoupler at the top of the base 1142 come into contact with the first optocoupler barrier, the first optocoupler barrier can generate a signal, telling that the magnet 101 is in the first position. When the magnet support member 114 moves along the linear guide rails 1141 to make the first optocoupler at the bottom of the base 1142 come into contact with the first optocoupler barrier, the first optocoupler barrier can generate a signal, telling that the magnet 101 is in the second position.

It should be noted that in other embodiments, the magnet driving mechanism 110 may be configured to drive the magnet 101 to move in other directions, for example, in the X-axis direction and/or in the Y-axis direction. In some embodiments, a plurality of magnet driving mechanisms 110 may be provided, which are used to drive the magnet 101 to move in different directions (e.g., the X-axis direction, the Y-axis direction, and the Z-axis direction).

In some embodiments, the mixing assembly 12 may adopt an electric motor driving mode (e.g., electrical transmission driving or shaking table electromagnetic driving) or any other feasible driving modes (such as hydraulic driving, or pneumatic driving).

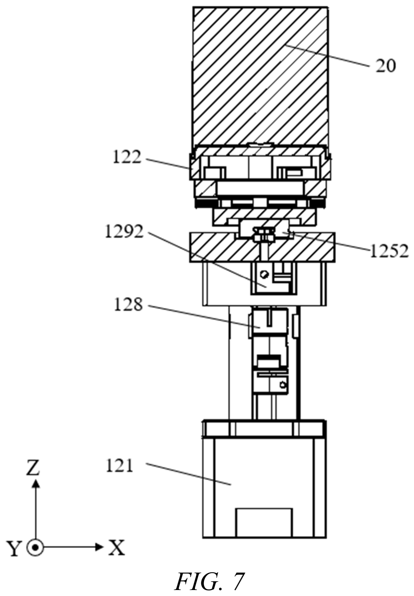

As shown in Figs. 1 and 5-7, in some embodiments, the mixing assembly 12 may include a second driving motor 121 and a mixing platform 122, the mixing platform 122 being driven by the second driving motor 121 to shake or vibrate. In some embodiments, the processing box 20 may be mounted on the mixing platform 122. For example, the processing box 20 may be manually mounted onto or removed from the mixing platform 122. The second driving motor 121 in operation can drive the mixing platform 122 to shake or vibrate, causing the processing box 20 thereon to shake or vibrate, thereby mixing the sample in the processing box 20. For example, the processing box 20 moves rapidly in the X-axis direction and the Y-axis direction in the horizontal plane (i.e., the X-Y plane) such that the sample in the processing box 20 is mixed.

In some embodiments, the mixing platform 122 may include an eccentric shaft 1221, and the second driving motor 121 may be drivingly connected to the eccentric shaft 1221. When the processing box 20 is mounted on the mixing platform 122, the eccentric shaft 1221 can be driven by the second driving motor 121 to rotate, and the rotation of the eccentric shaft 1221 then can drive the mixing platform 122 to shake or vibrate, thereby mixing the sample in the processing box 20.

By way of example only, the mixing assembly 12 may include a first mixing bearing 1231, a second mixing bearing 1232, a third mixing bearing 1233, a first direction adapter plate 1241, a second direction adapter plate 1242, a first mixing guide rail 1251, a second mixing guide rail 1252, a motor fixing plate 1211, and a support plate 126. The first direction adapter plate 1241, the second direction adapter plate 1242, and the support plate 126 are parallel to one another. In the third direction (i.e., the Z-axis direction), the mixing platform 122 is located above the first direction adapter plate 1241, the second direction adapter plate 1242 is located below the first direction adapter plate 1241, and the support plate 126 is located below the second direction adapter plate 1242. The first direction adapter plate 1241 is connected to the second direction adapter plate 1242 via the first mixing guide rail 1251, and the second direction adapter plate 1242 is connected to the support plate 126 via the second mixing guide rail 1252. The first mixing guide rail 1251 may be perpendicular to the second mixing guide rail 1252, the first mixing guide rail 1251 may extend in the second direction (i.e., the X-axis direction), and the second mixing guide rail 1252 may extend in the first direction (i.e., the Y-axis direction). The motor fixing plate 1211 may be connected to the underside of the support plate 126, and the second driving motor 121 may be connected to the motor fixing plate 1211. One end of the eccentric shaft 1221 is connected to the second driving motor 121, and the other end thereof passes through the support plate 126 and the second direction adapter plate 1242 and is connected to the first direction adapter plate 1241 via the first mixing bearing 1231. The eccentric shaft 1221 is connected to the second direction adapter plate 1242 via the second mixing bearing 1232, and to the support plate 126 via the third mixing bearing 1233. When the second driving motor 121 drives the eccentric shaft 1221 to move, the eccentric shaft 1221 may drive the second direction adapter plate 1242 to move along the second mixing guide rail 1252 relative to the support plate 126, and drive the first direction adapter plate 1241 to move along the first mixing guide rail 1251 relative to the second direction adapter plate 1242, thereby driving the mixing platform 122 to vibrate in the first and second directions to mix the sample.

In some embodiments, the second driving motor 121 may be directly fixed to the motor fixing plate 1211. In some embodiments, the second driving motor 121 may be fixed to the motor fixing plate 1211 via a connecting shaft 127. In some embodiments, the eccentric shaft 1221 may be directly connected to a rotary output shaft of the second driving motor 121. In some embodiments, the mixing assembly 12 may include a coupling 128, and the eccentric shaft 1221 may be connected to the rotary output shaft of the second driving motor 121 via the coupling 128.

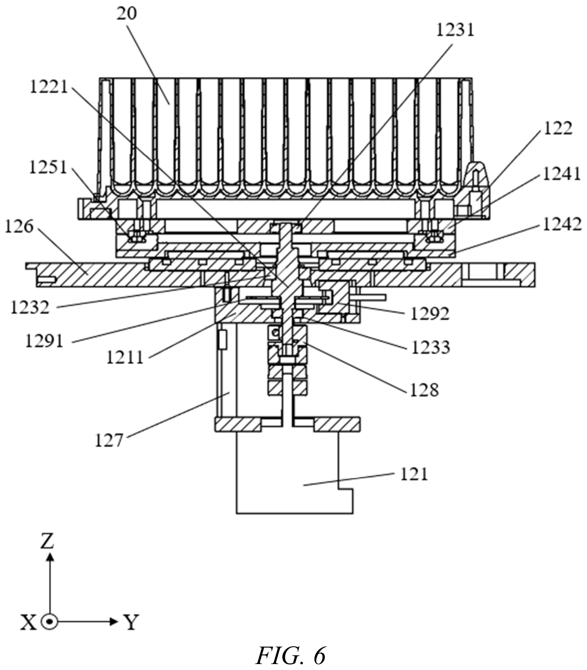

In some embodiments, as shown in Fig. 6, the eccentric shaft 1221 is provided with a second optocoupler barrier 1291, and the motor fixing plate 1211 is provided with a second optocoupler 1292. As the eccentric shaft 1221 rotates, the second optocoupler barrier 1291 is driven to rotate. When the second optocoupler barrier 1291 moves to the position of the second optocoupler 1292, it can be determined that the eccentric shaft 1221 is in the initial position, and accordingly the processing box 20 is also in the initial position. In some embodiments, the processing box in the initial position is closer to the magnet 101. In some cases, determining whether the processing box 20 is in the initial position by providing the second optocoupler barrier 1291 and the second optocoupler 1292 can, on the one hand, assist in positioning the pipetting device when aspirating liquid from the processing box 20 and, on the other hand, place the processing box 20 in the initial position so that it is closer to the magnet 101 when magnetic attraction is required.

The processing device for a sample provided in the present description may have the following beneficial effects, including, but not limited to: (1) The processing device provided in the present description can attract the magnetic beads conjugated to the impurities or the target biological substance in the processing box, thereby facilitating the transfer of the remaining solution with a pipetting device and the separation of the impurities or the target biological substance from the solution. In addition, the processing device can mix the mixture in the processing box to improve the efficiency and effectiveness of the extraction and/or purification of the target biological substance. (2) Driven by the magnet driving mechanism, the magnet can retract below the upper side surface of the magnetic attraction and mixing mechanism when there is no need to perform magnetic attraction on the sample, and extend above the upper side surface of the magnetic attraction and mixing mechanism when there is a need to perform magnetic attraction on the sample, so that the processing device can meet the various requirements of the different steps of extraction and/or purification of the target biological substance, thereby improving the applicability. (3) In order to prevent the sample in the other set of processing chambers from being affected, only one of the two sets of processing chambers is used to process the sample at the same moment. (4) The processing device can be used in combination with an extraction apparatus for a sample to extract and/or purify a target biological substance in the sample, which can significantly reduce the workload of experimenters and shorten the work time. Moreover, the processing device is mechanically automated throughout its work, with high consistency in repeated operations and better overall extraction and/or purification results.

The above is merely preferred embodiments of the present description, which, however, are not intended to limit the present description, and any modifications, equivalent replacements, improvements, etc. made within the spirit and principle of the present description should fall within the scope of protection of the present description.

## Claims

1. A processing device for a sample, **characterized by** comprising:
a magnetic attraction and mixing mechanism, comprising a mixing assembly and a magnetic assembly for respectively performing mixing and magnetic attraction on the sample; and
a processing box comprising two sets of processing chambers.

2. The device according to claim 1, **characterized in that** the magnetic assembly comprises a magnet disposed on at least one side of the mixing assembly, and the processing box is disposed on the mixing assembly.

3. The device according to claim 2, **characterized in that** the magnet comprises a first magnet disposed on one side of the mixing assembly.

4. The device according to claim 3, **characterized in that** the magnet further comprises a second magnet disposed on the other side of the mixing assembly.

5. The device according to claim 4, **characterized in that** the processing box is disposed between the first magnet and the second magnet, and the two sets of processing chambers are arranged in the same direction as the first magnet and the second magnet.

6. The device according to claim 2, **characterized in that** the magnetic assembly further comprises a magnet driving mechanism for driving the magnet to perform a lifting or lowering movement.

7. The device according to claim 6, **characterized in that** the magnet driving mechanism comprises a first driving motor, a lead screw and a linkage, wherein the lead screw is drivingly connected to the first driving motor, one end of the linkage is hinged to the lead screw and the other end of the linkage is directly or indirectly hinged to the magnet, and the magnet is driven by the first driving motor to extend above or retract below an upper side surface of the magnetic attraction and mixing mechanism.

8. The device according to claim 7, **characterized in that** the magnetic assembly further comprises a magnet support member for fixing the magnet, the linkage being hinged to the magnet support member.

9. The device according to claim 1, **characterized in that** the mixing assembly comprises a second driving motor and a mixing platform, the mixing platform being driven by the second driving motor to shake or vibrate.

10. The device according to claim 9, **characterized in that** the mixing platform comprises an eccentric shaft to which the second driving motor is drivingly connected.
